# EUROPÄISCHE PATENTANMELDUNG

(11) **EP 0 784 997 A2**
(43) Veröffentlichungstag der Anmeldung: **23.07.1997**
(21) Anmeldenummer: 96100740.8
(22) Anmeldetag: 19.01.1996
(51) Int. Cl.: A61N 5/06

(54) **Einrichtung zur Behandlung von Hautzonen**

(71) Anmelder: Koblinger, Gerald, Ing., A-5142 Eggelsberg (AT)
(72) Erfinder: Gucher, Alfred, A-5020 Salzburg (AT)

(57) **Zusammenfassung**

Im wesentlichen handelt es sich bei dieser Erfindung um eine Einrichtung zur Behandlung bestimmter Hautzonen mittels verschiedener Therapiemaßnahmen in Form eines Gerätes, dadurch gekennzeichnet, daß ein mit seinem freien, offenen Ende auf die zu behandelnde Hautzone (1) dicht aufsetzbares, glockenförmiges Gefäß (2) vorgesehen ist, in dessen gegenüberliegenden Ende, eine getrennte Ausgänge aufweisende Einheit (3 oder 3a) zur Applikation von Unterdruck, Farblicht und vorzugsweise vernebelten Wirkstoffen eingesetzt bzw. einsetzbar ist, wobei die Applikationseinheit (3 oder 3a) über voneinander getrennte Versorgungsleitungen und zwar einem Vakuumschlauch (10) und zwei zum Erzeugen und Regeln des Wirkstoffnebels dienenden,als Schlauchleitungen (11 und 11a) ausgebildete Steuerleitungen für Druckluft beziehungsweise einem Vakuumschlauch (10) und einem Lichtleiter (7) mit drei unabhängigen Versorgungseinheiten und zwar eine Einrichtung (4) zur regelbaren Erzeugung von Unterdruck, einer wählbare Farben abgebenden Farblichtquelle (5) und einer die Schlauchleitungen mit Druckluft speisenden Wirkstoffsteuereinheit (6), lösbar verbunden bzw. einzeln verbindbar ist, sodaß die einzelnen Therapiemaßnahmen (Unterdruck-, Farblicht- und Wirkstoffapplikation) entweder gleichzeitig oder getrennt,oder beliebig kombiniert anwendbar sind und daß ferner weitere mit der Applikationseinheit (3 oder 3a) verbindbare,glockenförmige Gefäße verschiedener Bauart und Größe vorgesehen sind.

## Beschreibung

Die Erfindung betrifft eine Einrichtung zur Behandlung bestimmter Hautzonen mittels verschiedener Therapiemaßnahmen in Form eines Gerätes.

Im besonderen handelt es sich bei diesen Therapiemaßnahmen um die Applikation von Unterdruck,Farblicht und Wirkstoffen auf die zu behandelnde Hautzone.
Diese Therapieformen,bzw. die dafür verwendbaren Einrichtungen,sind einzeln bereits lange Zeit in Anwendung,wie ein dicht auf die zu behandelnde Körperoberfläche aufsetzbares Gefäß,ab jetzt als Glocke bezeichnet,welches mit einer Einrichtung zur Erzeugung von Unterdruck verbunden ist und unter dem Ausdruck "Schröpfglas" allgemein bekannt ist.
Des weiteren ist z.B. aus der DE-A1-3301802 bekannt, in diesen Glocken Lichtquellen mit optischen Gittern und Filtern zu Bestrahlungszwecken, zu montieren.
Dadurch entstehen jedoch,konstruktionsbedingt Nachteile, wie z.B. die Unhandlichkeit der Glocke durch große Aufßenabmessungen, da alle Komponenten darin eingebaut sind, oder bei einer konstruktiv aufwendigen Verkleinerung der Glocke kommt es durch die schlechte Wärmeabfuhr zur Temparaturerhöhung und dadurch zu thermischen Problemen an der zu bestrahlenden Oberfläche; als Beispiel sei blaues Licht angeführt, welches ein kaltes Licht ist und, um seine volle therapeutische Wirkung zu entfalten, nicht mit einer Temperaturerhöhung an der bestrahlten Oberfläche verbunden sein darf.
Die vorliegende Erfindung löst dieses Problem dadurch, daß ein mit seinem freien; offenen Ende auf die zu behandelnde Hautzone (1) dicht aufsetzbares, glockenförmiges Gefäß (2) vorgesehen ist, in dessen gegenüberliegenden Ende, eine getrennte Ausgänge aufweisende Einheit (3 oder 3a) zur Applikation von Unterdruck, Farblicht und vorzugsweise vernebelten Wirkstoffen eingesetzt bzw. einsetzbar ist, wobei die Applikationseinheit (3 bzw. 3a) über voneinander getrennte Versorgungsleitungen und zwar einem Vakuumschlauch (10) und zwei zum Erzeugen und Regeln des Wirkstoffnebels dienenden Schlauchleitungen, ausgebildete Steuerleitungen für Druckluft (11 u. 11a),beziehungsweise einem Vakuumschlauch (10) und einem Lichtleiter (7) mit drei unabhängigen Versorgungseinheiten und zwar eine Einrichtung (4) zur regelbaren Erzeugung von Unterdruck, einer wählbare Farben abgebenden Farblichtquelle (5) und einer,die Schlauchleitungen mit Druckluft speisenden Wirkstoffsteuereinheit (6), lösbar verbunden bzw. einzeln verbindbar ist, sodaß die einzelnen Therapiemaßnahmen (Unterdruck -, Farblicht- und Wirkstoffapplikation) entweder gleichzeitig oder getrennt, oder beliebig kombiniert anwendbar sind und daß ferner weitere mit der Applikationseinheit (3 u. 3a) verbindbare, glockenförmige Gefäße verschiedener Bauart und Größe vorgesehen sind.
Durch die Verwendung von Lichtleitern wird nur das Licht selbst übertragen, aber keine Wärme. Weiters ermöglicht diese Konstruktion es, die glockenförmigen Gefäße in den Abmessungen klein zu halten und vom Anwender gegen andere Gefäße selbständig austauschen zu lassen.
Die erfindungsgemäße Einrichtung unterscheidet sich im Bezug auf die Farblicht-Therapie weiters von bisherigen Entwicklungen (DD-A1-219676) dadurch, daß nur Licht bestimmter Wellenlängen übertragen wird.Um die erwünschte Wirkung bei der therapeutischen Anwendung zu erzielen,sind Lichtquellen im Bereich der Spektralfarben möglichst ohne störenden UV-Anteil erforderlich, zu diesem Zweck werden, um die erwünschte Wirkung zu erzielen, erfindungsgemäß spezielle Farbfilterscheiben verwendet und der unerwünschte UV-Anteil von einem weiteren Filter zum größtmöglichen Teil absorbiert.

Bei Einrichtungen zur Wirkstofftherapie sind verschiedene Einrichtungen bereits bekannt, wie z.B. das Verdampfen von Wirkstoffen, welche eingeatmet werden. Die Erfindung liegt in diesem Fall auch darin, daß durch die Kombination der Unterdruck - und Wirkstoffapplikation in der Glocke, eine weit größere Wirksamkeit auf der zu behandelnden Körperoberfläche entfaltet wird, wobei noch zusätzlich die Farblicht-Therapie eingesetzt werden kann.
Daher kann gesagt werden, daß mit einer Kombination dieser Therapiemaßnahmen gemäß der vorliegenden Erfindung sich starke Wirkungen hinsichtlich der Erzeugung von Reizen und anderen Vorgängen in bestimmten Hautzonen des Menschen erzielen lassen.
Die Wirkung der Therapie beschränkt sich jedoch nicht nur auf Bindegewebsschichten, Blut und Lymphe, sondern erregt die Nerven in der Haut und die darunter liegenden Weichteile wie z.B. Muskeln und Sehnen. Sie wirkt auf tiefliegende Nervenstämme und bringt dadurch vielfältige sich auf die inneren Organe übertragende Reize hervor.
Die Therapie in ihren verschiedenen Formen bedingt reaktive Hyperämie, fördert den Zufluß und Abfluß der Gewebssäfte, erhöht die Permeablilität der Kapillarwände, tonisiert innere Organe, fördert die Ausscheidung,
unterstützt die Blutzirkulation bei insuffizienten Kreislauf, übt Rückwirkung auf das allgemeine Kraftgefühl, auf Ermüdungszustände, Stimmung und Psyche aus.

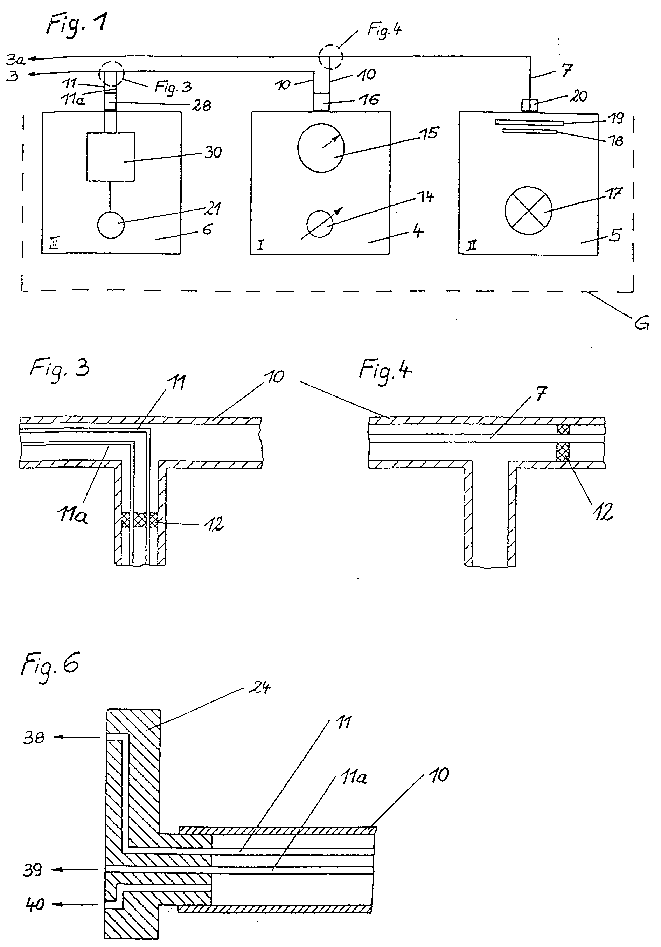

Ein Ausführungsbeispiel des Erfindungsgegenstandes wird nachfolgend in den Figuren 1 - 6 erläutert.

Die Figur 1 zeigt die erfindungsgemäße Einrichtung zur kombinierten Unterdruck-, Farblicht- und Wirkstofftherapie ohne glockenformiges Gefäß (2),Applikationseinheit (3 bzw. 3a),wobei die drei Versorgungseinheiten (4,5,6) je nach Ausführung in ein gemeinsames Gehäuse (G),wie auch in seperaten Modulen (I,II,III) eingebaut sein können.

In Figur 2 wird das auf die zu behandelnde Hautzone (1) dicht aufsetzbare und somit mit seinem Innenraum hermetisch abschließbare glockenförmige Gefäß (2),nachfolgend Glocke bezeichnet,gezeigt,wobei die Glocke (2) mit der Unterdruck- und Wirkstoffapplikations-Einheit (3) verbunden ist.

Diese Applikations-Einheit (3 u. 3a) ist wiederum mit der Einrichtung zur Erzeugung von Unterdruck (4) der Farblicht-Quelle (5) und der Wirkstoffsteuer-Einheit (6) durch mit für die einzelnen Medien geeignete Versorgungsleitungen verbunden.

Die Versorgungsleitungen sind als Lichtleiter (7) und als drei weitere Schlauchleitungen (10, 11 und 11a) ausgebildet.

Die Figur 3 zeigt eine mögliche Ausführungsform der Unterdruck u. Wirkstoffapplikationsleitung,wobei der Vakuumschlauch (10) als Ummantelung für die anderen Leitungen (11 u.11a) ausgeführt ist.

Die Figur 4 zeigt eine mögliche Ausführungsform der Unterdruck u. Farblicht -Therapieleitung,wobei der Vakuumschlauch (10) als Ummantelung für den Lichtleiter (7) ausgeführt ist. Die Lichtleiter (7) können dann lose im verbleibenden Raum zwischen Vakuumschlauch (10) geführt werden, wobei bei der Aufteilung der Versorgungsleitungen zu den einzelnen Versorgungseinheiten (4,5 u.6) dann eine Dichtung (12) zur Aufrechterhaltung des Unterdruckes vorgesehen ist.

Aus diesem Grund sind auch mehrere Dichtungen (12'u.12'') zwischen Glocke (2) und Applikations-Einheit (3 u. 3a) sowie in der Applikationseinheit (3) vorgesehen.

Da Unterdruckabbau aber zur leichteren Handhabung der Glocke für therapeutische Zwecke notwendig ist, ist die Glocke (2) mit einer Öffnung (13) versehen, welche von Hand geschlossen werden kann.

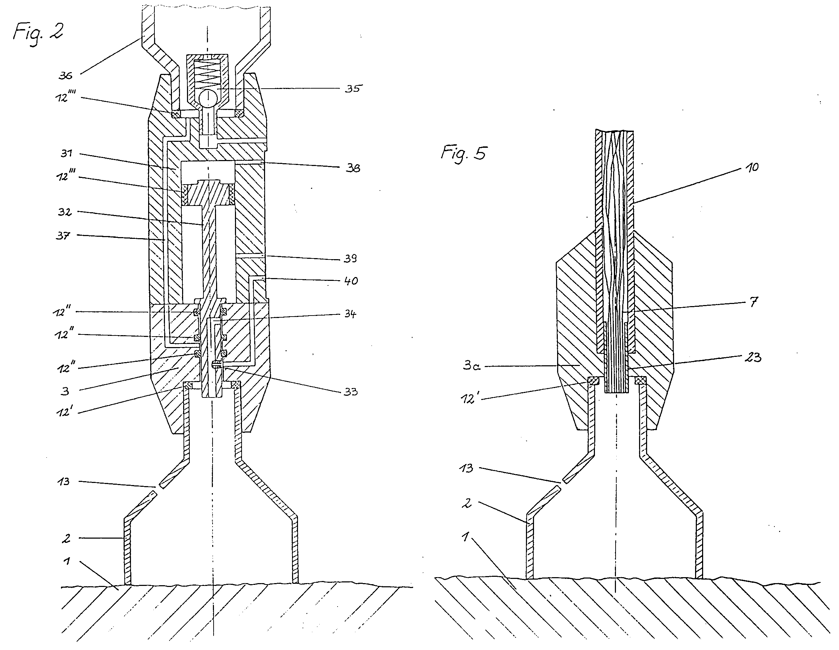

In Figur 5 wird die Einmündung des Lichtleiters in die Applikationseinheit (3a) verdeutlicht.Die Lichtleiter werden in die Hülse (23) eingepreßt und somit fixiert und gebündelt,wobei sich der Unterdruck in der verbleibenden Fläche zwischen den einzelnen Lichtleitern ungehindert ausbreiten kann.Die Hülse (23) wird mit einem Ende mit der Applikationseinheit (3a) verbunden; über das andere Ende wird der Vakuumschlauch (10) geschoben, wodurch die Dichtheit des Systems gewährleistet wird.

In Figur 6 wird die Verbindung der einzelnen Leitungen, bei der Unterdruck und Wirkstoffapplikation, mit der Applikationseinheit (3) verdeutlicht.Im wesentlichen handelt es sich hierbei um einen Verteilerblock (24), welcher die Verbindungen zwischen Schlauchleitung (11) und Steuerluftkanal (38), beziehungsweise Schlauchleitung (11a) und Steuerluftkanal (39), beziehungsweise Vakuumschlauch (10) und Vakuumkanal (40) herstellt.

Der Unterdruck in der Glocke (2) wird mittels der regelbaren Einrichtung (4) bestimmt, wobei eine mögliche Ausführungsform ein von Hand zu verstellendes Drosselventil (14) mit einer visuellen Anzeigeeinheit (15) ist. Die Verbindung (16) zur Transportleitung (10) kann lösbar ausgeführt sein.

In der Farblicht-Quelle (5) ist eine Lichtquelle (17) installiert, über UV-Sperrfilter (18) und Farbfilter (19) gelangt das Licht in den mittels lösbarer Verbindung (20) befestigten Lichtleiter (7), der zur Glocke (2) führt.

In der Wirkstoffsteuer-Einheit (6) wird Druckluft geregelt, welche für den Vernebelungsvorgang der Wirkstoffe (ätherische Öle) in der Aufbereitungseinheit (Fig.2) notwendig ist. Der dafür nötige Druck wird durch eine Pumpe (21), die sich in der Wirkstoffsteuer-Einheit (6) befindet, erzeugt. Mittels lösbarem Anschluß (28) und der als Schlauch ausgeführten Wirkstoffsteuer-Leitungen (11 u. 11a) wird wiederum die Verbindung zur Applikationseinheit (3) hergestellt.

### Funktionsbeschreibung:

Um den gewünschten therapeutischen Effekt zu erzielen, muß der Unterdruck in der Glocke (2), mittels Drosselventil (14) an der Einrichtung zur Unterdruckerzeugung (4), an der jeweiligen Hautoberfläche (1) angepaßt werden. Durch Öffnen oder Verschließen der Öffnung (13) an der Glocke (2) kann der Anwender die Dauer des an der Haut (1) anliegenden Unterdruckes bestimmen.

Das Farblicht wirkt ununterbrochen auf die Hautoberfläche in der gewünschten Farbe (Wellenlänge) ein.

In der Applikationseinheit (3) wird der Wirkstoff vernebelt. Nach dem Betätigen eines Tasters, steuert ein Magnetventil (30) die von der Pumpe (21) erzeugte Druckluft und wird in weiterer Folge durch die Wirkstoffsteuerleitungen (11 u. 11a) zum Pneumatikzylinder (31) geleitet. Der Wirkstoffsteuerkolben (32) mit dem Kolbenring (12''') bewegt sich nun für einen kurzen Augenblick nach oben; in diesem Moment ist der Düsenluftkanal (34) unter Druck. Die Düsenluft strömt nun entlang des Kanales bis zur Düse (33), welche in diesem Zustand direkt am Wirkstoff anliegt. Durch die vorbeiströmende Luft entsteht eine Sogwirkung in der Düse (33), wodurch der Wirkstoff in den Luftstrom gelangt und vernebelt wird. Der Wirkstoffkanal (37) leitet den Wirkstoff vom Wirkstoffbehälter (36) zur Applikationseinheit (3). Um den im Wirkstoffbehälter (36) auftretenden Unterdruck zu kompensieren befindet sich in diesem das Lüftungsventil (35);zwischen Wirkstoffbehälter (36) und Applikationseinheit (3)
befindet sich ebenfalls eine Ringdichtung (12'''').
Dieser Vernebelungsvorgang wird entweder durch einen Fußtaster, oder durch einen Taster, welcher sich an der Wirkstoffsteuereinheit befindet ausgelöst. Die Dauer der Vernebelung wird durch eine in der Wirkstoffsteuereinheit integrierten Elektronik geregelt, unabhängig davon, wielange man einen dieser beiden Taster auslöst. Mittels eines Reglers an der Wirkstoffsteuereinheit kann die Länge des Vernebelungsvorganges, an die verschieden voluminösen Glocken und an die unterschiedlichen Unterdrücke der einzelnen Behandlungen so angepaßt werden, damit gewährleistet ist, daß in den Glocken immer ein Unterdruck besteht.

Die beschriebene Konstruktion der beliebigen Anschließbarkeit der Versorgungseinheiten (4,5 u.6) ermöglicht es den Anwendern auch die einzelnen Einheiten (4,5 u.6) jeweils alleine zu Betreiben, wie auch wahlweise miteinander zu kombinieren, oder sie gleichzeitig zu betreiben.

## Patentansprüche

1. Einrichtung zur Behandlung bestimmter Hautzonen mittels verschiedener Therapiemaßnahmen in Form eines Gerätes, dadurch gekennzeichnet, daß ein mit seinem freien, offenen Ende auf die zu behandelnde Hautzone (1) dicht aufsetzbares, glockenförmiges Gefäß (2) vorgesehen ist, in dessen gegenüberliegenden Ende, eine getrennte Ausgänge aufweisende Einheit (3 oder 3a) zur Applikation von Unterdruck, Farblicht und vorzugsweise vernebelten Wirkstoffen eingesetzt bzw. einsetzbar ist, wobei die Applikationseinheit (3 oder 3a) über voneinander getrennte Versorgungsleitungen und zwar einem Vakuumschlauch (10) und zwei zum Erzeugen und Regeln des Wirkstoffnebels dienenden,als Schlauchleitungen (11 und 11a) ausgebildete Steuerleitungen für Druckluft beziehungsweise einem Vakuumschlauch (10) und einem Lichtleiter (7) mit drei unabhängigen Versorgungseinheiten und zwar eine Einrichtung (4) zur regelbaren Erzeugung von Unterdruck, einer wählbare Farben abgebenden Farblichtquelle (5) und einer die Schlauchleitungen mit Druckluft speisenden Wirkstoffsteuereinheit (6), lösbar verbunden bzw. einzeln verbindbar ist, sodaß die einzelnen Therapiemaßnahmen (Unterdruck-, Farblicht- und Wirkstoffapplikation) entweder gleichzeitig oder getrennt,oder beliebig kombiniert anwendbar sind und daß ferner weitere mit der Applikationseinheit (3 oder 3a) verbindbare,glockenförmige Gefäße verschiedener Bauart und Größe vorgesehen sind.

2. Einrichtung nach Anspruch 1, dadurch gekennzeichnet, daß das glockenförmige Gefäß (2) mit einer vom Anwender verschließbaren Öffnung (13) versehen ist.

3. Einrichtung nach Anspruch 1 oder 2, dadurch gekennzeichnet, daß die Farblichtquelle (5) neben einer Lichtquelle (17) und einer Farbfilterscheibe (18) einen UV-Sperrfilter (19) aufweist.

4. Einrichtung nach Anspruch 1, 2 oder 3, dadurch gekennzeichnet, daß in der Appilkationseinheit (3) Mittel (32,34,12'') vorgesehen sind,welche nur während der durch Druckluftzufuhr erfolgenden Vernebelung des Wirkstoffes die Steuerleitungen (11 u. 11a) für Druckluft mit dem Innenraum des Glockenförmigen Gefäßes (2) verbinden,um den im Inneren der Glocke (2) herrschenden Unterdruck im wesentlichen aufrecht zu erhalten.

5. Einrichtung nach Anspruch 1,2,3 oder 4, dadurch gekennzeichnet, daß ein Wirkstoffbehälter (36) direkt auf die Wirkstoffapplikationseinheit (3) dicht aufsetzbar und mittels eines Wirkstoffkanales (37) mit der Vernebelungseinheit verbunden ist.
